# EUROPEAN PATENT APPLICATION

(11) **EP 4 455 279 A1**
(43) Date of publication of application: **30.10.2024**
(21) Application number: 21968652.4
(22) Date of filing: 24.12.2021
(51) Int. Cl.: C12N 9/12, C12N 15/54, C12Q 1/686

(54) **DNA POLYMERASE MUTANT AND USE THEREOF**

(71) Applicant: BGI Shenzhen, Shenzhen, Guangdong 518083 (CN)
(72) Inventor: ZHANG, Xiaohong, Shenzhen, Guangdong 518083 (CN); LAN, Xi, Shenzhen, Guangdong 518083 (CN); XIE, Qingqing, Shenzhen, Guangdong 518083 (CN); XI, Feng, Shenzhen, Guangdong 518083 (CN); ZHENG, Yue, Shenzhen, Guangdong 518083 (CN); DONG, Yuliang, Shenzhen, Guangdong 518083 (CN); ZHANG, Wenwei, Shenzhen, Guangdong 518083 (CN)
(74) Representative: Gill Jennings & Every LLP
(86) International application number: PCT/CN2021/141114
(87) International publication number: WO 2023/115517

(57) **Abstract**

The present invention relates to the field of biotechnology, in particular to a DNA polymerase mutant and the use thereof. Compared with a wild type, the mutant provided in the present invention has a significantly improved DNA polymerization activity, significantly improved affinity to DNA and amplification uniformity; and has good effects in the amplification of multiplex PCR, high-GC templates and complex templates. A hot-start-version DNA polymerase is further prepared by means of using antibodies or chemical modifications, wherein the yield of the DNA polymerase is significantly improved, while the primer dimer is significantly decreased. The prepared mutant hot-start DNA polymerase can be applied to PCR amplification of multiplex PCR high-GC templates and complex templates.

## Description

### FIELD

The present invention relates to the field of biotechnology, and specifically relates to a DNA polymerase mutant and the use thereof.

### BACKGROUND

Polymerase chain reaction (PCR) is a molecular biology technique used to amplify specific DNA fragments. It can be used not only for basic research such as gene separation, cloning and nucleic acid sequence analysis, but also for disease diagnosis, sequencing or any place where DNA and RNA exist. DNA polymerase, template, primers and dNTPs are needed in PCR reaction, among which DNA polymerase plays a vital role in PCR reaction, which not only affects the efficiency of the amplification but also affects the accuracy of the amplification.

A general PCR process is divided into three steps, namely denaturation, annealing and extension. In the denaturation stage, the hydrogen bonds of the double-stranded DNA are broken at high temperatures (90°C to 98°C) to generate single-stranded DNAs. In the annealing stage, the temperature is reduced to the annealing temperature (50°C to 65°C), allowing the primers to bind to the template and form partial double strands. In the extension stage, the reaction temperature is adjusted to the extension temperature of the DNA polymerase (70°C to 75°C), under the action of DNA polymerase, using dNTPs as the substrate, the complementary DNA strand of the template is synthesized from the 3' end of the primer along the 5' to 3' direction.

In a nucleic acid sequence, the proportion of guanine (G) and cytosine (C) is referred to the GC content. In base complementary pairing, two hydrogen bonds are formed between A and T, while three hydrogen bonds are formed between G and C. Therefore, DNA templates with high GC content are more prone to form stable secondary structure, and require higher energy for denaturation. To overcome the interaction between G and C, the most common method is to rely on PCR additives to assist in the denaturation of DNA, such as DMSO and single-stranded DNA-binding protein (SSB). These reagents typically reduce the Tm of the primers by reducing the renaturation of DNA, thereby improving the efficiency of PCR on GC-rich template. DNA polymerase with high synthesis capacity is more beneficial for PCR amplification of a template with high GC content due to their strong binding ability with the template. Additionally, using a hot-start DNA polymerase for PCR can improve the yield of PCR products with high GC content, reduce nonspecific amplification, and reduce the formation of primer dimers. Chemical hot-start DNA polymerase is produced by treating DNA polymerase with chemical reagents such as citraconic anhydride, dimethyl maleic anhydride, formaldehyde, etc., to allow lysine become lysine anhydride. After becoming anhydride, the activity of DNA polymerase is inhibited at room temperature, and gradually released when the temperature is increased.

In addition, multiplex PCR is often used in various detection scenarios such as clinical diagnosis. Multiplex PCR involves adding more than two pairs of primers to the same PCR reaction system, allowing the simultaneous amplification of various nucleic acid fragments, which is highly efficient, systematic and economic. Due to the amplification of various fragments, multiplex PCR not only requires DNA polymerases with high activity but also amplification uniformity, that is, the all different fragments in the same system should be amplified well.

Despite there are many schemes to modify Taq enzyme in current technology, simply improving the polymerase activity of DNA polymerases inevitably leads to a series of problems such as poor amplification uniformity. Therefore, there is no Taq enzyme that has three properties at the same time, which is, high DNA polymerization activity, capability to amplifying complex templates and high GC templates, and can also be used for multiplex PCR.

### SUMMARY

In view of this, the present invention provides a DNA polymerase mutant and use thereof.

The present invention provides a DNA polymerase mutant comprising at least one mutation selected from the group consisting of K56Q, A61T and E507K, and the amino acid sequence of the wild type DNA polymerase is set forth in SEQ ID NO: 1.

In some embodiments, the DNA polymerase mutant comprises the mutation selected from the group consisting of K56Q, A61T and E507K, wherein the amino acid sequence of the DNA polymerase mutant is set forth in SEQ ID NO: 2.

The present invention also provides a hot-start DNA polymerase, wherein the hot-start DNA polymerase is the DNA polymerase mutant binding with a Taq antibody, or the hot-start DNA polymerase is the DNA polymerase mutant chemically modified by a reagent.

The reagent for chemical modification in the present invention is citraconic anhydride.

The present invention further provides a nucleic acid encoding the DNA polymerase mutant.

In some particular embodiments, the sequence encoding the mutant comprising K56Q, A61T and E507K mutations is set forth in SEQ ID NO: 3.

The present invention further provides an expression vector comprising the nucleic acid of the present invention.

The backbone of the expression vector of the present invention is a pET vector. In some embodiments, the backbone vector is pET-28a vector.

The present invention further provides a host cell transformed or transfected with the expression vector.

In some embodiments, the host cell is a prokaryotic cell. In some particular embodiments, the host cell is *Escherichia coli* DH5α.

A method of producing the DNA polymerase mutant of the present invention comprises culturing the host cell and inducing the expression of the DNA polymerase mutant.

Use of the DNA polymerase mutant, the hot-start DNA polymerase, the nucleic acid, the expression vector, the host cell or the DNA polymerase mutant produced by the method of the present invention in the manufacture of a reagent for PCR reaction.

The present invention further provides a reagent for PCR reaction, which comprises the DNA polymerase mutant, the hot-start DNA polymerase or the product produced by the method.

The present invention further provides a PCR method, comprising performing amplification by using the mutant of the present invention, the hot-start DNA polymerase of the present invention or the DNA polymerase mutant produced by the method of the present invention as Taq enzyme.

The PCR reactions described in the present invention include multiplex PCR, PCR with high GC content template, PCR with samples containing inhibitor, fast PCR, and PCR with fluorescent reagents.

Compared with the wild type DNA polymerase, the DNA polymerase mutant provided by the present invention exhibits significantly improved DNA polymerase activity, affinity with DNA, and amplification uniformity, demonstrating superior performance in multiplex PCR and the amplification with a high GC content template. A hot-start DNA polymerase is produced by antibody modification or chemical modification, and the yield of PCR products is significantly increased while the formation of primer dimer is significantly reduced. The hot-start DNA polymerase mutant can be used for multiplex PCR, PCR amplification with a high GC content template and a complex template.

### BRIEF DESCRIPTION OF DRAWINGS

FIG. 1 shows the results of the multiplex PCR (8-plex) using a Taq DNA polymerase and a mutant thereof, respectively.
FIG. 2 shows the results of the multiplex PCR (5-plex) using a Taq DNA polymerase and a mutant thereof, respectively.
FIG. 3 shows the results of the amplification of a high GC fragment using a Taq DNA polymerase and a mutant thereof, respectively.
FIG. 4 shows the results of the amplification of complex template using a Taq DNA polymerase and a mutant thereof, respectively.
FIG. 5 shows the results of the multiplex PCR (8-plex) using a hot-start Taq DNA polymerase and a mutant thereof, respectively.
FIG. 6 shows the results of the multiplex PCR (5-plex) using a hot-start Taq DNA polymerase and a mutant thereof, respectively.

### DETAILED DESCRIPTION

The present invention provides a DNA polymerase mutant and use thereof. The content of the present invention can be used as a reference by those skilled in the art to achieve suitable modifications and improvements of process parameters. It should be noted that all similar replacements and modifications are obvious to those skilled in the art, and they are deemed to be included in the present invention. The method and the application of the present invention have been described through the preferred embodiments, and it is obvious that the method and application described herein may be changed or appropriately modified and combined to realize and apply the technology of the present invention by those skilled in the art without departing from the content, spirit and scope of the present invention.

Unless defined otherwise, all technical and scientific terms used herein have the same meaning as understood by those ordinarily skilled in the art. For definitions and terms in the art, professionals may in particular refer to Current Protocols in Molecular Biology (Ausubel). The abbreviations for amino acid residues are the standard three-letter and/or one-letter codes used in the art to refer to one of the 20 common L-amino acids.

The amino acid sequence of the wild-type DNA polymerase is set forth in SEQ ID NO: 1 and the length is 832. The amino acid sequence of SEQ ID NO: 1 is:














.

Compared to the wild-type DNA polymerase shown in SEQ ID NO: 1, the DNA polymerase mutant described in the present invention comprises one or more amino acid residue changes. The DNA polymerase mutant might be used for multiplex PCR, PCR amplification on a high GC template or a complex template. Compared to the wild-type DNA polymerase, the DNA polymerase activity, affinity with DNA, and amplification uniformity of the DNA polymerase mutant are significantly improved, showing better effect on multiplex PCR and the PCR amplification on a GC-rich template.

In some embodiments, the mutation of the DNA polymerase mutant is K56Q.

Alternatively, the mutation of the DNA polymerase mutant is A61T.

Alternatively, the mutation of the DNA polymerase mutant is E507K.

Alternatively, the mutations of the DNA polymerase mutant are K56Q and A61T.

Alternatively, the mutations of the DNA polymerase mutant are K56Q and E507K.

Alternatively, the mutations of the DNA polymerase mutant are A61T and E507K.

Alternatively, the mutations of the DNA polymerase mutant are K56Q, A61T and E507K, and the amino acid sequence is set forth in SEQ ID NO: 2 as follows:

MRGMLPLFEPKGRVLLVDGHHLAYRTFHALKGLTTSRGEPVQAVYGFAKSLLKALQEDG
DTVIVVFDAKAPSFRHEAYGGYKAGRAPTPEDFPRQLALIKELVDLLGLARLEVPGYEAD
DVLASLAKKAEKEGYEVRILTADKDLYQLLSDRIHVLHPEGYLITPAWLWEKYGLRPDQ
WADYRALTGDESDNLPGVKGIGEKTARKLLEEWGSLEALLKNLDRLKPAIREKILAHMD
DLKLSWDLAKVRTDLPLEVDFAKRREPDRERLRAFLERLEFGSLLHEFGLLESPKALEEA
PWPPPEGAFVGFVLSRKEPMWADLLALAAARGGRVHRAPEPYKALRDLKEARGLLAKD
LSVLALREGLGLPPGDDPMLLAYLLDPSNTTPEGVARRYGGEWTEEAGERAALSERLFA
NLWGRLEGEERLLWLYREVERPLSAVLAHMEATGVRLDVAYLRALSLEVAEEIARLEAEV
FRLAGHPFNLNSRDQLERVLFDELGLPAIGKTKKTGKRSTSAAVLEALREAHPIVEKILQY
RELTKLKSTYIDPLPDLIHPRTGRLHTRFNQTATATGRLSSSDPNLQNIPVRTPLGQRIRRAF
IAEEGWLLVALDYSQIELRVLAHLSGDENLIRVFQEGRDIHTETASWMFGVPREAVDPLM
RRAAKTINFGVLYGMSAHRLSQELAIPYEEAQAFIERYFQSFPKVRAWIEKTLEEGRRRG
YVETLFGRRRYVPDLEARVKSVREAAERMAFNMPVQGTAADLMKLAMVKLFPRLEEM
GARMLLQVHDELVLEAPKERAEAVARLAKEVMEGVYPLAVPLEVEVGIGEDWLSAKE.

The nucleic acid sequence encoding SEQ ID NO: 2 is set forth in SEQ ID NO: 3as follows:

atgcgtggcatgctgccgctgttcgagccgaagggtcgtgtgctgctggttgacggccaccacctggcgtaccgtacctttcacgcgctgaa
gggtctgaccaccagccgtggcgaaccggtgcaggcggtttatggtttcgcgaaaagcctgctgaaggcgctgcaagaggacggcgata
cggtgatcgtggttttcgatgcgaaggcgccgagctttcgtcacgaagcgtacggtggctataaagcgggtcgtgcgccgaccccggagg
actttccgcgtcaactggcgctgattaaagaactggttgatctgctgggtctggcgcgtctggaagtgccgggctacgaagcggacgatgtt
ctggcgagcctggcgaagaaagcggagaaggaaggttacgaagtgcgtatcctgaccgcggacaaagatctgtatcagctgctgagcga
ccgtatccacgttctgcacccggagggttatctgattaccccggcgtggctgtgggaaaagtacggcctgcgtccggaccaatgggcggat
tatcgtgcgctgaccggtgacgagagcgataacctgccgggcgttaaaggtattggcgaaaaaaccgcgcgtaagctgctggaggaatgg
ggtagcctggaagcgctgctgaaaaacctggatcgtctgaagccggcgatccgtgagaaaattctggcgcacatggacgatctgaagctg
agctgggacctggcgaaagtgcgtaccgacctgccgctggaagtggatttcgcgaagcgtcgtgagccggatcgtgaacgtctgcgtgcg
ttcctggagcgtctggaatttggtagcctgctgcacgagtttggcctgctggaaagcccgaaggcgctggaggaagcgccgtggccgccg
ccagagggtgcgttcgtgggctttgttctgagccgtaaagaaccgatgtgggcggacctgctggcgctggcggcggcgcgtggtggccgt
gtgcaccgtgcgccggagccgtacaaggcgctgcgtgacctgaaagaagcgcgtggtctgctggcgaaagatctgagcgttctggcgct
gcgcgaaggtctgggtctgccgccgggtgacgatccgatgctgctggcgtacctgctggatccgagcaacaccaccccggagggtgtgg
cgcgtcgttatggtggcgaatggaccgaggaagcgggcgagcgtgcggcgctgagcgaacgtctgttcgcgaacctgtggggtcgtctg
gagggcgaggaacgtctgctgtggctgtaccgtgaggtggaacgtccgctgagcgcggttctggcgcacatggaagcgaccggtgtgcg
tctggacgttgcgtatctgcgtgcgctgagcctggaagtggcggaggaaatcgcgcgtctggaggcggaagttttccgtctggcgggccac
ccgtttaacctgaacagccgtgaccagctggagcgtgttctgtttgatgaactgggtctgccggcgattggcaagaccaaaaaaaccggcaa
gcgtagcaccagcgcggcggtgctggaggcgctgcgtgaagcgcacccgatcgttgagaagattctgcagtaccgtgaactgaccaagc
tgaaaagcacctatatcgacccgctgccggatctgattcacccgcgtaccggtcgtctgcacacccgtttcaaccaaaccgcgaccgcgac
cggtcgtctgagcagcagcgacccgaacctgcagaacatcccggtgcgtaccccgctgggtcaacgtatccgtcgtgcgtttattgcggag
gaaggctggctgctggttgcgctggattacagccagattgagctgcgtgttctggcgcacctgagcggtgacgaaaacctgatccgtgttttc
caagagggccgtgatattcacaccgaaaccgcgagctggatgtttggtgtgccgcgtgaggcggttgacccgctgatgcgtcgtgcggcg
aagaccatcaacttcggtgtgctgtatggcatgagcgcgcaccgtctgagccaggagctggcgatcccgtacgaggaagcgcaggcgttt
attgaacgttatttccaaagctttccgaaggttcgtgcgtggattgagaaaaccctggaggaaggtcgtcgtcgtggttacgtggaaaccctgt
tcggtcgtcgtcgttacgttccggatctggaggcgcgtgtgaaaagcgttcgtgaggcggcggaacgtatggcgttcaacatgccggtgca
gggtaccgcggcggacctgatgaaactggcgatggttaagctgtttccgcgtctggaggaaatgggcgcgcgtatgctgctgcaagtgcac
gatgagctggttctggaagcgccgaaggagcgtgcggaagcggtggcgcgtctggcgaaagaggtgatggaaggtgtttacccgctggc
ggttccgctggaagttgaggtgggtatcggcgaggactggctgagcgcgaaagaataa.

In the present invention, for the convenience of purification, a 6×His tag is connected to the N-terminal of the DNA polymerase mutant.

In some embodiments of the present invention, the hot-start DNA polymerase is the mutant binding with a Taq antibody. The method for producing the hot-start DNA polymerase is: mixing the Taq DNA polymerase mutant, Taq antibody and Taq storage buffer, and reacting at room temperature to produce the hot-start DNA polymerase. Among them, the volume ratio of the Taq DNA polymerase mutant, the Taq antibody, and the Taq storage buffer is 2:1:1. The Taq storage buffer contains: water, 20mM Tris, 100mM KCl, 0.1mM EDTA, 1mM DTT, 0.5% Tween 20, 0.5% NP-40 and 50% glycerol. The reaction time at room temperature is 10 minutes.

In some other embodiments of the present invention, the hot-start DNA polymerase is the mutant modified with citraconic anhydride. The method for producing the hot-start DNA polymerase comprises steps of: mixing a DNA polymerase mutant solution with a citraconic anhydride solution and incubating, and then dialyzing the mixture with Taq storage buffer to obtain the hot-start DNA polymerase. The buffer for DNA polymerase mutant is Tris buffer, and the concentration of the DNA polymerase mutant is 0.5 mg/mL. The concentration of the Tris buffer is 40 mM, pH 9.5. The concentration of the citraconic anhydride solution is 1M. The volume ratio of the DNA polymerase mutant solution to the citraconic anhydride solution is 100:1. The incubation condition is at 42°C for 4 hours. The Taq storage buffer contains: water, 20mM Tris, 100mM KCl, 0.1mM EDTA, 1mM DTT, 0.5% Tween 20, 0.5% NP-40 and 50% glycerol. The reaction time at room temperature is 10 minutes.

In the present invention, the hot-start DNA polymerase is further produced by using antibody modification or chemical modification. When using the hot-start DNA polymerase for PCR, the yield of the amplification products is significantly increased, while the production of the primer dimer is significantly decreased. The hot-start DNA polymerase mutant can be used for multiplex PCR, PCR amplification on high GC template and a complex template.

The nucleic acid encoding the DNA polymerase mutant described in the present invention may be DNA, RNA or PNA. The nucleic acid might include nucleotide sequences having different functions, such as coding region and non-coding region, for example, regulatory sequences (e.g., promoter or transcription terminator). The DNA may be cDNA, genomic DNA, or artificially synthesized DNA. In the plasmid vectors constructed in the present invention, the nucleic acid is DNA. The DNA described in the present invention may be in the form of single-stranded DNA or double-stranded DNA. The nucleic acid may be linear or circular in topology. The nucleic acid may be, for example, a part or a fragment of a vector (such as an expression vector or cloning vector). The nucleic acid may be obtained directly from a natural source or prepared by recombinant, enzymatic method, or chemical techniques. In the nucleic acid encoding the DNA polymerase mutant of the present invention, the codon encoding the Q amino acid at position 56 is CAA, the codon encoding the T amino acid at position 61 is ACG, and the codon encoding the K amino acid at position 507 is AAA.

In the present invention, eukaryotic or prokaryotic plasmid vectors are used as the backbone for the expression vectors, which contain the nucleic acid encoding the DNA polymerase mutant. In some embodiments, the backbone of the expression vector is selected from T vector, pGEX series vector, pET series vector, etc. The pET series vector include: pET-3a, pET-5a, pET-9a, pET-11a, pET-12a, pET-14b, pET-15b, pET-16b, pET-17b, pET-19b, pET-20b, pET-21, pET-21a, pET-22b, pET-23, pET-23a, pET-24, pET-24a, pET-25b, pET-26b, pET-27b, pET-28a, pET-28b, pET-29a, pET-30a, pET-31b and pET-32a. The method for constructing the expression vector is not particularly limited by the present invention, and conventional methods in the field are used in the embodiments.

The host cells of the present invention are transformed or transfected with the expression vector. In the present invention, the expression vector may be transformed into a host cell alone or together with other plasmid vectors. The host cells may be animal cells, fungal cells or prokaryotic cells. In some embodiments, the host cell is a prokaryotic cell, and the prokaryotic cell is *Escherichia coli.* In some particular embodiments, the host cell is selected from the group consisting of *Escherichia coli* DH5α, BL21, Rosetta and BL21 (DE3). The method for constructing the host cell is not limited herein, and may be produced by conventional methods in the field. In the particular embodiment, the method for producing the host cell comprises: transforming the expression vector into the *E.coli* DH5α competent cells to obtain the host cells.

The method for producing the mutant described in the present invention comprises culturing the host cells described in the present invention and inducing the expression of the mutant. The culture medium for the culture is LB medium containing kanamycin. The culture condition is at 37 °C and 200 rpm for 8-12 hours, until the OD600 reaches 0.6-0.8. The inducer for induction expression is IPTG, and the concentration of the inducer is 0.5 mM. The induction condition is at 37 °C and 200 rpm for 4 hours. After obtaining the culture comprising the mutant, the bacteria are collected, and the Taq DNA polymerase is purified with Ni affinity column.

The reagents for PCR provided by the present invention include the mutants described in present invention, 10×PCR buffer, 10mM dNTP and water.

The present invention relates to DNA polymerase and a PCR kit. The DNA polymerase is a Taq DNA polymerase mutant. In the present invention, a plasmid comprising the coding sequence of the Taq DNA polymerase mutant is constructed by genetic engineering, and it is transformed into *E. Coli* for the expression of the Taq DNA polymerase. Then, the proteins are purified by using techniques such as affinity chromatography and ion exchange chromatography, thereby obtaining the Taq DNA polymerase mutant. Specifically, plasmids comprising coding sequences of different Taq DNA polymerase mutants are constructed by site-directed mutation PCR, see Example 1 for details. Then, the plasmids comprising coding sequences of different Taq DNA polymerase mutants are transformed into *E. Coli* for culturing and the protein expression is induced. Finally, the DNA polymerase is purified by affinity chromatography and ion exchange chromatography to obtain the Taq DNA polymerases with different mutations, see Example 2 for details. Through Taq antibody modification or chemical modification methods, the hot-start Taq DNA polymerase mutants are generated, see Example 3 for details.

In addition, the present invention further provides a method for detecting the activity of DNA polymerase. This method comprises using M13 ssDNA bound with a primer as a template-primer complex for DNA chain extension in the presence of DNA polymerase to obtain a double-stranded DNA product by extension. Fluorescent molecules are incorporated into the reaction products and the activity of DNA polymerase is calculated by detecting the amount of double-stranded DNA. The Taq DNA polymerase mutant with improved polymerase activity is screened by this method, see Example 4 for details.

On the other hand, in the present invention, the Taq DNA polymerase mutants are used for PCR of GC-rich and complex templates, and for multiplex PCR for simultaneous amplification of multiple genes. The Taq DNA polymerase mutants are screened by electrophoresis analysis to select a mutant suitable for amplification with high GC template, PCR with complex template and multiplex PCR, see Example 5, Example 6 and Example 7 for details.

Finally, it is found that the hot-start Taq DNA polymerase mutant could further increase the yield and reduce the production of primer dimer multiplex PCR see Example 8 for details.

The materials used in the present invention are all commercially available and could be purchased from the market. The present invention is further illustrated below in conjunction with examples.

### Example 1 Construction of expression plasmids of Taq DNA polymerase and mutants

The construction steps are as follows.

### (1) Construction of expression plasmid of wild-type Taq DNA polymerase

The amino acid sequence of wild-type Taq DNA polymerase was set forth in SEQ ID NO: 1.

The expression plasmid pET-28a-Taq comprising the coding sequence of wild-type Taq DNA polymerase was purchased from GenScript Technology Co., Ltd. The N-terminal of the amino acid sequence was fused with His tag (6xHis) to facilitate the purification of the protein.

### (2) Construction of expression plasmid of Taq DNA polymerase mutant

The forward and reverse primers for mutations were designed based on the mutation sites screened by rational design, and pfu DNA polymerase was used for extension. The specific reaction system was shown in Table 1.

**Table 1 PCR system for constructing expression plasmid of Taq DNA polymerase mutant**

| Reaction components | Volume (µL) |
|---|---|
| 10×pfu buffer (containing MgSO₄) | 2.5 |
| 2.5 mM dNTPs | 2 |
| 10 µM forward / reverse primer | 0.7 |
| pfu DNA polymerase | 0.5 |
| 50 ng/µL template (pET-28a-Taq or mutant) | 1 |
| H₂O | 17.6 |

The reaction conditions for PCR were shown in Table 2.

**Table 2 PCR conditions for constructing expression plasmid of Taq DNA polymerase mutant**

| Temperature | time | cycle number |
|---|---|---|
| 95 °C | 5 min | 1 |
| 95 °C | 30 s | 19 |
| 53 °C | 30 s | |
| 68 °C | 8 min | |
| 68 °C | 10 min | 1 |
| 4 °C | ∞ | 1 |

After the completion to the reaction, 1 µL of DpnI was added to the system for digestion of the PCR product at 37°C for 1 hour. Subsequently, 5 µL of the product after digestion was transformed into *E.Coli* DH5α competent cells, and the transformation results were observed. Single clones were then picked from the plate for culturing and then the plasmids were extracted. Finally, the obtained mutants were analyzed by sequence alignment.

The mutants constructed in the present invention were as follows: Taq Mut1 (K56Q), Taq Mut2 (A61T), Taq Mut3 (E507K) and Taq Mut4 (K56Q/A61T/E507K).

### Example 2 Induction, expression and purification of Taq DNA polymerase and mutants

Wild-type Taq DNA polymerase and mutants, which carried 6xHis tag at the N-terminal, were expressed by pET-28a vector, and the proteins were purified by 6xHis tag using Ni column affinity purification.

The plasmids of the wild-type and mutants were transformed into BL21 competent cells (purchased from TransGen Biotech Co., Ltd.). Single clones were picked and cultured in 5 mL of LB medium containing kanamycin (50 µg/mL) overnight at 37°C, 200 rpm. At the next day, the cultures were transferred to 1500 mL fresh LB medium containing kanamycin (50 µg/mL) at a ratio of 1:100, then incubated at 37°C, 200 rpm until the OD600 reached 0.6-0.8. Inducer IPTG was added and the final concentration was 0.5 mM. The cultures were further incubated at 37°C, 200 rpm for 4 hours to induce the expression of proteins. After induction, the bacteria were collected by centrifugation at 8000 rpm for 10 minutes.

The bacteria precipitate was collected and resuspended with Ni-column affinity solution A (50 mM Tris, 500 mM NaCl, 0.5% Triton X-100, 5% glycerol, 10 mM imidazole, pH 7.8). The cells were broken by ultrasound sonication in an ice-water bath, and the conditions of ultrasonication were as follows: the diameter of ultrasonic amplitude transformer was 10 mm, the ultrasonic intensity was 40%, of ultrasonication was performed 2 seconds followed by 3 seconds interval, for a total of 30 minutes. The mixture of broken bacteria was put into a water bath at 75°C for 30min and then centrifuged at 13,000 rpm, 4°C for 30 minutes, and the supernatant was collected.

The supernatant sample obtained in the above step was subjected to affinity purification, as follows: according to the AKTA operation flow, the working pump and system were washed with filtered and degassed MilliQ water, then 5 ml pre-packed His trap FF was loaded at a flow rate of 0.5mL/min. The column was washed with 5 CV of H₂O, and then equilibrated with SCV Taq DNA polymerase Ni column affinity solution A. The supernatant sample was loaded on the chromatographic column at 5 mL/min. After loading, the column was washed with 10 CV Taq DNA polymerase Ni column affinity solution A. The proteins were eluted by gradient elution with Taq DNA polymerase Ni column affinity solution B (50mM Tris, 500mM NaCl, 0.5% Triton X-100, 5% glycerol, 500mM imidazole, pH 7.8) accounting for 0-50% of the eluate (10 CV) and the target proteins were collected.

The sample obtained by affinity purification was diluted 10 times with Taq dilution buffer (50mM Tris, 0.5% Triton X-100, 5% glycerol, pH7.8) and then subjected to anion exchange chromatography. The specific steps were as follows: according to the AKTA operation flow, the working pump and system were washed with filtered and degassed MilliQ water, then 5 mL pre-packed His trap FF was loaded at a flow rate of 0.5mL/min. The column was washed with 5 CV of H₂O, and then equilibrated with SCV Taq DNA polymerase ionic buffer A (20mM Tris, 50mM NaCl, 5% glycerol, pH7.8). The diluted sample was loaded on the chromatographic column at a speed of 5 mL/min. After loading, the column was washed with 10 CV Taq DNA polymerase ionic buffer A, and then linear gradient eluted with Taq DNA polymerase ionic buffer B (20mM Tris, 1M NaCl, 5% glycerol, pH7.8) accounting for 0-100% of the eluate (15 CV), and finally the target protein was collected.

The purified target proteins were dialyzed and stored at -20°C for subsequent determination and analysis.

### Example 3 Production of hot-start Taq DNA polymerase and hot-start Taq DNA polymerase mutants

Hot-start PCR can prevent non-specific amplification, amplify low concentration targets and the reaction system could be prepared at room temperature. Hot-start DNA polymerase is the main means to carry out hot-start PCR. Hot-start Taq DNA polymerase or mutants could be prepared by binding with Taq antibody. Or the chemically modified hot-start Taq DNA polymerase could be obtained by treating purified Taq DNA polymerase or mutants and Taq antibody with citraconic anhydride.

The production process of antibody-modified hot-start Taq DNA polymerase or mutants were as follows: 0.2 mg/mL of purified Taq DNA polymerase or mutants, Taq antibody, and Taq storage buffer (20 mM Tris, 100 mM KCl, 0.1 mM EDTA, 1 mM DTT, 0.5% Tween 20, 0.5% NP-40, 50% glycerol) were mixed in a ratio of 2:1:1, and the mixture was left at room temperature for 10 minutes to complete the production of the antibody-modified hot-start DNA polymerase. The obtained antibody-modified hot-start Taq DNA polymerase or mutants could be stored at -20°C.

The production process of chemically modified hot-start Taq DNA polymerase or mutants were as follows: 0.2 mg/mL purified Taq DNA polymerase or mutants were concentrated to 0.5 mg/mL with a 30K ultrafilter and the solution was changed to 40 mM Tris (pH9.5). Then, 0.5 mg/mL Taq DNA polymerase or mutants were mixed with 1M citraconic anhydride in the ratio of 100:1, and incubated at 42°C for 4 hours. After the mixture was dialyzed with Taq storage buffer (20 mM Tris, 100 mM KCl, 0.1 mM EDTA, 1 mM DTT, 0.5% Tween20, 0.5% NP-40, 50% glycerol), the sample was diluted to 0.1 mg/mL to complete the production of chemically modified hot-start DNA polymerase. The obtained chemically modified hot-start Taq DNA polymerase or chemically modified hot-start Taq DNA polymerase mutants could be stored at -20°C.

### Example 4 Determination and analysis of polymerization activity of Taq DNA polymerase and mutants thereof

The polymerization activity of purified wild-type Taq DNA polymerase and mutants was determined.

In the activity assay, M13 ssDNA bound with primer was used as the template-primer complex, and the DNA chain was extended under the action of DNA polymerase to generate the double-stranded DNA product. Fluorescent molecules were incorporated into the reaction products, and the activity of DNA polymerase was calculated by measuring the amount of double-stranded DNA. The sequence of the primer bound with the template was: 5'-agcgaacctcccgacttgcgggagg-3'. The formula of 10× PCR buffer used in the present invention was as follows: 100 mM Tris, 500 mMKCl, 15 mM MgCl₂, 25% glycerol and 0.5 mg/mL BSA.

The reaction system for measuring Taq DNA polymerase activity was shown in Table 3.

**Table 3 System for measuring polymerization activity of Taq DNA polymerase**

| Reaction component | Volume (µL) |
|---|---|
| 10× PCR buffer | 2.5 |
| 50 nM M13 ssDNA template-primer complex | 2 |
| 10 mM dNTP | 1 |
| 4 ng/µL Taq DNA polymerase or mutant | 1 |
| H₂O | 18.5 |

The reaction system was prepared and put into a PCR machine, the reaction was carried out at 72°C for 5 minutes, and then 0.5 µL of 0. 5M EDTA was added to terminate the reaction. The amount of double-stranded DNA was measured by Qubit dsDNA HS Assay Kit, and the polymerization activity of the mutant relative to the wild type was obtained by calculation.

The results of activity determination showed that the polymerization activities of Taq Mut1, Taq Mut2, Taq Mut3 and Taq Mut4 were 0.47, 1.01, 4.37 and 3.84 times of that of the wild-type Taq, respectively.

### Example 5 Using Taq DNA polymerase or mutants for multiplex PCR

The purified wild-type Taq DNA polymerase and mutants thereof were used for the 8-plex amplification of human housekeeping genes and 5-plex amplification of mouse housekeeping genes.

### (1) 8-plex amplification of human housekeeping genes

The template for 8-plex amplification was human genomic DNA, and the amplification primers and the length of target fragments were shown in Table 4.

**Table 4. Primers and length of target fragments of the 8-plex amplification of human housekeeping genes**

| Gene | Primer (5'-3') | Length of target fragment (bp) |
|---|---|---|
| CYB5A | ggcaacgcttagactctgtgtg | 998 |
| | ctgcccttggcctaactaacct | |
| PRPH | gttcctcaagaagctgcacgag | 744 |
| | cgttagactctggatctggcgt | |
| GABARAPL2 | ccagccaattcatgagtcggtg | 595 |
| | cctgacaactcgcaagtagcac | |
| ACTG1 | gctcaatggggtacttcagggt | 485 |
| | gtggacgttacgtaaaaggccc | |
| NDUFA7 | tgctctggatgtgaagatgcca | 405 |
| | ttccaggtaaatccagcccagg | |
| UQCRC1 | cagccagtcagcatcatccaac | 315 |
| | gaaagccggattgcggtaacat | |
| MYC | ggatagctctgcaaggggagag | 214 |
| | tcgtcgcagtagaaatacggct | |
| MIF | agaagtcaggcacgtagctcag | 113 |
| | ggcacgttggtgtttacgatga | |

The PCR reaction system for 8-plex amplification of human housekeeping genes was shown in Table 5.

**Table 5. Reaction system for 8-plex amplification of human housekeeping genes**

| Reaction component | Volume (µL) |
|---|---|
| 10× PCR buffer | 5 |
| 10 mM dNTPs | 1 |
| 5 µM primer mixture | 2 |
| 10 ng/µL human gDNA | 2 |
| 0.1 mg/mL Taq DNA polymerase or mutant | 0.5 |
| H₂O | 39.5 |

The PCR conditions for 8-plex amplification of human housekeeping genes were shown in Table 6.

**Table 6. 8-plex amplification conditions of human housekeeping genes**

| Temperature | Time | Cycle number |
|---|---|---|
| 95 °C | 3 min | 1 |
| 95 °C | 10 s | 30 |
| 64 °C | 30 s | |
| 72 °C | 1 min | |
| 72 °C | 5 min | 1 |
| 4 °C | ∞ | 1 |

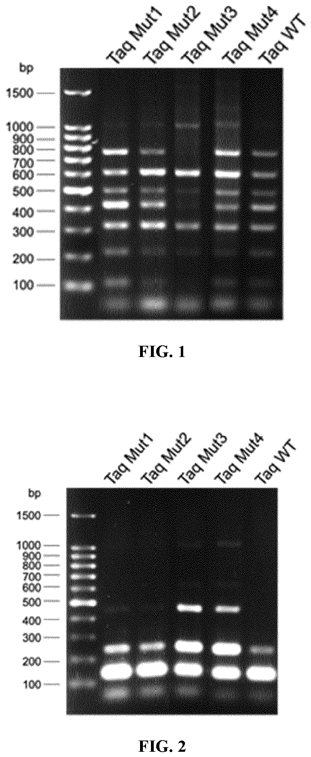

After the 8-plex amplification of human housekeeping gene completed, 10 µL of 6× DNA loading buffer (60 mM Tris, 60 mM EDTA, 60% glycerol, orange G) was added to the reaction system and mixed well, and then electrophoresis analysis was performed with 1.3% agarose gel. The results were shown in FIG. 1.

Although Taq Mut1 and Taq Mut2 could amplify 8 target fragments, the yield of different bands was quite different, and there was obvious amplification bias. Similarly, Taq Mut3 could amplify a long fragment, but it also has serious preference. Taq Mut4 could amplify 8 target fragments, and the yield of each was quite uniform, and the amplification bias was significantly reduced.

### (2) 5-plex amplification of mouse housekeeping genes

The template for 5-plex amplification of mouse housekeeping genes was the cDNA of Chinese hamster ovary (CHO) cells, which was prepared by extracting the total RNA from CHO cells as a template, and performing reverse transcription with the reverse transcriptase Superscript II Reverse Transcriptase using a primer containing polyA to obtain the cDNA of CHO.

The primers and the length of target fragments of 5-plex amplification of mouse housekeeping genes were shown in Table 7.

**Table 7. Primers and length of target fragments of the 5-plex amplification of mouse housekeeping genes**

| Gene | Primer | Length of target fragment (bp) |
|---|---|---|
| Rpl13a | acggaaggaaaaggccaagatgcac | 143 |
| | attgggttcacaccaggagtccgtt | |
| GAPDH | agcctcgtcccgtagacaaaatggt | 243 |
| | agatggtgatgggcttcccgttgat | |
| Pgk1 | cggtgttgccaaaatgtcgctttcc | 448 |
| | ggccggctcagctttaaccttgttt | |
| B2m | accggagaatgggaagccgaacata | 665 |
| | acagggttgggggtgagaattgcta | |
| β-Actin | gagcacagcttctttgcagctcctt | 994 |
| | tcctgtcagcaatgcctgggtacat | |

The PCR reaction system for 5-plex amplification of mouse housekeeping genes was shown in Table 8.

**Table 8. The reaction system for 5-plex amplification of mouse housekeeping genes**

| Reaction component | Volume (µL) |
|---|---|
| 10× PCR buffer | 5 |
| 10 mM dNTPs | 1 |
| 5 µM primer mixture | 2 |
| 10 ng/µL CHO cDNA | 2 |
| 0.1 mg/mL Taq DNA polymeraseor mutant | 0.5 |
| H₂O | 39.5 |

The PCR conditions for 5-plex amplification of mouse housekeeping genes were shown in Table 9.

**Table 9. The 5-plex amplification conditions of mouse housekeeping genes**

| Temperature | Time | Cycle number |
|---|---|---|
| 95 °C | 3 min | 1 |
| 95 °C | 10 s | 30 |
| 55 °C | 30 s | |
| 72 °C | 1 min | |
| 72 °C | 5 min | 1 |
| 4 °C | ∞ | 1 |

After the 5-plex amplification of mouse housekeeping genes completed, 10 µL of 6× DNA loading buffer (60 mM Tris, 60 mM EDTA, 60% glycerol, orange G) was added to the reaction system and mixed well, and then electrophoresis analysis was performed with 1.3% agarose gel. The results were shown in FIG. 2.

The results showed that Taq Mut1, Taq Mut2, Taq Mut3 and Taq Mut4 could amplify three or more targets, among them, Taq Mut4 showed the best performance, which could amplify five bands with relatively high yield, while the wild-type Taq DNA polymerase could only amplify 2 out of the 5 bands.

### Example 6 amplification of GC-rich fragments by Taq DNA polymerase or mutants thereof

The purified wild-type Taq DNA polymerase and mutants were tested by PCR on GC-rich template.

The human gDNA was used as the template and the GCH1 gene with 70% GC content was amplified. The sequence of upstream primer F was cggctcggagtgtgatctaagcaggt, and the sequence of downstream primer R was agaggtcgctgccacccaggaag, the fragment length was 697bp.

The PCR reaction system was shown in Table 10.

**Table 10 Reaction system of amplification of high GC content template**

| Reaction component | Volume (µL) |
|---|---|
| 10× PCR buffer | 5 |
| 10mM dNTP | 1 |
| 10µM primer F | 2 |
| 10µM primer R | 2 |
| 10ng/µL human gDNA | 2 |
| 0.1mg/mL Taq DNA polymerase or mutant | 0.5 |
| H₂O | 39.5 |

The PCR conditions were shown in Table 11.

**Table 11 Reaction conditions of amplification of high GC content template**

| Temperature | Time | Cycle number |
|---|---|---|
| 95 °C | 3 min | 1 |
| 95 °C | 30 s | 30 |
| 64 °C | 30 s | |
| 72 °C | 1 min | |
| 72 °C | 5 min | 1 |
| 4 °C | ∞ | 1 |

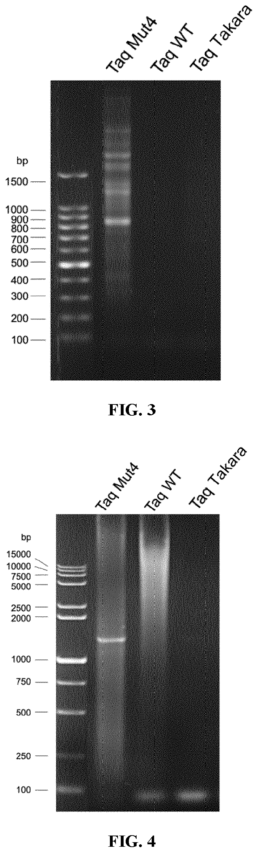

After the PCR completed, 10 µL of 6× DNA loading buffer (60 mM Tris, 60 mM EDTA, 60% glycerol, orange G) was added to the reaction system and mixed well, and then electrophoresis analysis was performed with 1.3% agarose gel. The results were shown in FIG. 3.

The results showed that Taq Mut4 could amplify the target fragment of 697bp, while the wild-type Taq DNA polymerase and Taq enzyme from Takara did not get amplification product.

### Example 7 amplification of complex template by Taq DNA polymerase and mutants

The purified wild-type Taq DNA polymerase and mutants were tested by PCR with complex template.

Human gDNA was used as a template in the test, and the β-globin gene, which is a complex template, was amplified at a low template amount of 10 ng. The sequence of upstream primer F was ttaggccttagcgggcttagac. The sequence of downstream primer R was ccaggatttttgatgggacacg. The length of the fragment was 1.3kb.

The PCR reaction system was as follows:

**Table 12 Reaction system of amplification with complex template**

| Reaction component | Volume (µL) |
|---|---|
| 10× PCR buffer | 5 |
| 10mM dNTP | 1 |
| 10µM primer F | 2 |
| 10µM primer R | 2 |
| 10ng/µL human gDNA | 1 |
| 0.1 mg/mL Taq DNA polymerase or mutant | 0.5 |
| H₂O | 39.5 |

The PCR conditions were as follows:

**Table 13 Reaction conditions of amplification with complex template**

| Temperature | Time | Cycle number |
|---|---|---|
| 95 °C | 3 min | 1 |
| 95 °C | 30 s | 30 |
| 60 °C | 30 s | |
| 72 °C | 1 min | |
| 72 °C | 5 min | 1 |
| 4 °C | ∞ | 1 |

After the PCR completed, 10 µL of 6× DNA loading buffer (60 mM Tris, 60 mM EDTA, 60% glycerol, orange G) was added to the product and thoroughly mixed, and then electrophoresis analysis was performed with 1.3% agarose gel. The specific results were shown in FIG. 4.

The results showed that the Taq Mut4 could amplify and generate a specific target fragment with a length of 1.3kb, while the product of the wild-type Taq DNA polymerase showed a smear phenomenon and no target band. The Taq enzyme of Takara produced a really weak target band and obvious primer dimers.

### Example 8 Multiplex PCR performed by using a hot-start Taq DNA polymerase or a hot-start Taq DNA polymerase mutant.

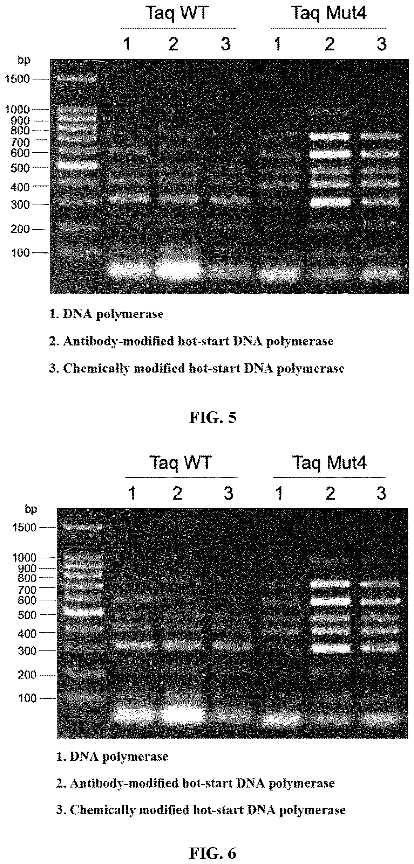

The purified wild-type Taq DNA polymerase, chemically modified hot-start wild-type Taq DNA polymerase, antibody-modified hot-start wild-type Taq DNA polymerase, Taq DNA polymerase mutant, chemically modified hot-start Taq DNA polymerase mutant and antibody-modified hot-start Taq DNA polymerase mutant were used for the test of 8-plex amplification of human housekeeping gene and 5-plex amplification of mouse housekeeping gene. The specific operation process was the same as that of Example 5, and the results were shown in FIGs 5 and 6.

The results showed that in the 8-plex PCR amplification system, 8 target fragments were successfully amplified by Taq Mut4, while only 7 out of the 8 fragments were amplified by the wild-type Taq DNA polymerase. Using chemically modified hot-start Taq Mut4 or antibody-modified hot-start Taq Mut4 could not only successfully amplified 8 target fragments, but also further improved the yield and reduced the generation of primer dimer.

Similarly, in the 5-plex PCR amplification system, five target fragments were successfully amplified by Taq Mut4, while only two out of the five fragments were amplified by the wild-type Taq DNA polymerase. Using chemically modified hot-start Taq Mut4 or antibody-modified hot-start Taq Mut4 could not only successfully amplified five target fragments, but also further improved the yield and reduced the generation of primer dimer.

The above is only the preferred embodiment of the present invention, and it should be pointed out that a person skilled in the art can make several improvements and embellishments without departing from the principle of the present invention, and these improvements and embellishments should also be regarded as the protection scope of the present invention.

## Claims

1. A DNA polymerase mutant comprising at least one mutation selected from the group consisting of K56Q, A61T and E507K, wherein the mutation is relative to a wild type DNA polymerase and the amino acid sequence of the wild type DNA polymerase is set forth in SEQ ID NO: 1.

2. The DNA polymerase mutant according to claim 1, wherein the amino acid sequence of the mutant is set forth in SEQ ID NO: 2.

3. A hot-start DNA polymerase, wherein the hot-start DNA polymerase is the DNA polymerase mutant according to claim 1 or 2 binding with a Taq antibody, or
the hot-start DNA polymerase is the DNA polymerase mutant according to claim 1 or 2 chemically modified by a reagent.

4. The hot-start DNA polymerase according to claim 3, wherein the reagent for chemical modification is citraconic anhydride.

5. A nucleic acid encoding the DNA polymerase mutant according to claim 1 or 2.

6. The nucleic acid according to claim 5, wherein the sequence of the nucleic acid is set forth in SEQ ID NO: 3.

7. An expression vector comprising the nucleic acid according to claim 5 or 6.

8. A host cell transformed or transfected with the expression vector according to claim 7.

9. A method of producing the DNA polymerase mutant according to claim 1 or 2, comprising culturing the host cell according to claim 8 and inducing the expression of the DNA polymerase mutant.

10. Use of the DNA polymerase mutant according to claim 1 or 2, the hot-start DNA polymerase according to claim 3 or 4, the nucleic acid according to claim 5 or 6, the expression vector according to claim 7, the host cell according to claim 8 or the DNA polymerase mutant produced by the method according to claim 9 in the manufacture of a reagent for PCR reaction.

11. A reagent for PCR reaction comprising the DNA polymerase mutant according to claim 1 or 2, the hot-start DNA polymerase according to claim 3 or 4 or the DNA polymerase mutant produced by the method according to claim 10.

12. A PCR method, comprising performing amplification by using the DNA polymerase mutant according to claim 1 or 2, the hot-start DNA polymerase according to claim 3 or 4 or the DNA polymerase mutant produced by the method according to claim 10 as Taq enzyme.
